(19) Europäisches Patentamt
European Patent Office
Office européen des brevets

(11) **EP 3 114 646 B1**

(12) **EUROPEAN PATENT SPECIFICATION**

(45) Date of publication and mention of the grant of the patent:
**29.08.2018 Bulletin 2018/35**

(21) Application number: **15709313.9**

(22) Date of filing: **04.03.2015**

(51) Int Cl.:
*G06T 7/00* (2017.01)     *G06T 7/11* (2017.01)
*G06T 7/12* (2017.01)     *G06T 7/136* (2017.01)
*G06T 7/149* (2017.01)

(86) International application number:
**PCT/GB2015/050620**

(87) International publication number:
**WO 2015/132585 (11.09.2015 Gazette 2015/36)**

(54) **APPARATUS AND METHOD FOR DETECTING MYOCARDIAL IRON CONTENT USING BLACK-BLOOD MAGNETIC RESONANCE DATA**

VORRICHTUNG UND VERFAHREN ZUR ERKENNUNG VON MYOKARDIALEM EISENGEHALT MITTELS DATEN DER SCHWARZBLUT-MAGNETRESONANZ

APPAREIL ET PROCÉDÉ DE DÉTECTION D'UNE TENEUR EN FER DU MYOCARDE EN UTILISANT DES DONNÉES DE RÉSONANCE MAGNÉTIQUE DE SANG NOIR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.03.2014 GB 201403877**

(43) Date of publication of application:
**11.01.2017 Bulletin 2017/02**

(73) Proprietor: **St. George's Hospital Medical School London SW17 0RE (GB)**

(72) Inventors:
• **HE, Taigang**
  **London**
  **Greater London HA4 8RY (GB)**
• **FENG, Yanqiu**
  **Guanzhou 510515 (CN)**

(74) Representative: **J A Kemp**
  **14 South Square**
  **Gray's Inn**
  **London WC1R 5JJ (GB)**

(56) References cited:
  **WO-A2-2006/134430     US-A1- 2004 153 128**

• QIAN ZHENG ET AL: "Automated interventricular septum segmentation for black-blood myocardial T2* measurement in thalassemia", JOURNAL OF MAGNETIC RESONANCE IMAGING, 27 May 2014 (2014-05-27), pages n/a-n/a, XP055185579, ISSN: 1053-1807, DOI: 10.1002/jmri.24662
• YANQIU FENG ET AL: "In vivo comparison of myocardial T1 with T2 and T2* in thalassaemia major", JOURNAL OF MAGNETIC RESONANCE IMAGING, vol. 38, no. 3, 31 January 2013 (2013-01-31), pages 588-593, XP055185581, ISSN: 1053-1807, DOI: 10.1002/jmri.24010
• Cmrtools: "Thalassaemiatools - T2* assessment for the heart and liver", , 23 July 2010 (2010-07-23), XP055185587, Retrieved from the Internet: URL:http://web.archive.org/web/20100723144 115/http://www.cmrtools.com/cmrTools/publi c/thalassaemiatools.pdf [retrieved on 2015-04-23]
• PHALLA OU ET AL: "Cardiac T2* measurements in patients with iron overload: a comparison of imaging parameters and analysis techniques", MAGNETIC RESONANCE IMAGING, ELSEVIER SCIENCE, TARRYTOWN, NY, US, vol. 30, no. 5, 3 December 2011 (2011-12-03), pages 641-648, XP028480071, ISSN: 0730-725X, DOI: 10.1016/J.MRI.2011.12.020 [retrieved on 2012-02-02]

- ZHANG YAONAN ET AL: "The shape analysis of ventricular septum based on medical images", 2013 IEEE INTERNATIONAL CONFERENCE ON MEDICAL IMAGING PHYSICS AND ENGINEERING, IEEE, 19 October 2013 (2013-10-19), pages 201-206, XP032625508, DOI: 10.1109/ICMIPE.2013.6864534 [retrieved on 2014-07-24]

- QIAN ZHENG ET AL: "Gaussian Regularizing CV Model Using Entropy and Neighborhood Information", WORLD CONGRESS ON MEDICAL PHYSICS AND BIOMEDICAL ENGINEERING MAY 26-31, 2012, BEIJING, CHINA IFMBE PROCEEDINGS,, vol. 39, 1 January 2013 (2013-01-01), pages 1832-1835, XP009183891,

**Description**

The present invention relates to methods and apparatus for detecting myocardial iron content, for example in thalassemia major patients, involving in particular an automated and reliable way approach for segmenting the intervectricular septum in black-blood myocardial T2* measurements.

Regular blood transfusion has greatly prolonged survival and improved the quality of life for patients with haematological conditions such as thalassemia major. However, multiple transfusion therapy can result in progressive accumulation of iron which can cause damage in many organs, particularly the liver, heart, and endocrine organs over time, ultimately leading to increased morbidity and mortality. Iron-induced cardiac dysfunction remains the leading cause of death in thalassemia patients. Chelation therapy can remove excessive tissue iron from the body and reduce the risk of organ failure in these patients.

It is important to be able to determine myocardial iron concentrations accurately and reliably to manage treatment and assess prognosis of patients.

Cardiovascular magnetic resonance (CMR) has been established as a non-invasive approach for detecting myocardial iron content (MIC) in various patients with iron overload. A single breath-hold bright-blood T2* method has been widely employed due to its speed, sensitivity, and wide availability (14-16). Compared with the bright-blood technique, the black-blood T2* technique has superior reproducibility because this technique avoids signal contamination from blood and shows a clearer definition of the myocardial/blood boundary due to suppressed blood signal and reduced imaging artifacts (17,18).

In routine clinical practice, a representative T2* value of a region-of-interest (ROI) is usually reported as a quantitative marker of MIC. T2* measurements are preferably performed in the region of interventricular septum (IS), as this region is least affected by severe susceptibility effects from epicardial fat, liver and lung tissues. The septum ROI is determined manually and may suffer from intra- and inter-observer variability due to the subjectivity of the operator and the uneven MIC in the radial direction. Manual determination of the ROI is also time-consuming and interrupts data processing of the CMR data.

YANQIU FENG et al.: "In vivo comparison of myocardial T1 with T2 and T2* in thalassaemia major", Journal of Magnetic Resonance Imaging, vol. 38, no. 3, 31 January 2013, pages 588-593, discloses a method in which myocardial T1 is compared against T2 and T2* in patients with thalassemia major (TM) for myocardial iron characterization. The method involves manual identification of the interventricular septum.

Cmrtools: "Thalassaemiatools - T2* assessment for the heart and liver", 23 July 2010, URL:http://web.archive.org/web/20100723144115/http://www.cmrtools.com/cmrTools/public /thalassaemiatools.pdf discloses a tool used to manually identify the interventricular septum in the method of YANQIU FENG et al. mentioned above.

PHALLA OU et al.: "Cardiac T2* measurements in patients with iron overload: a comparison of imaging parameters and analysis techniques", MAGNETIC RESONANCE IMAGING, ELSEVIER SCIENCE, TARRYTOWN, NY, US, vol. 30, no. 5, 3 December 2011, pages 641-648 describes a study examining the impact of different imaging parameters and data analysis techniques on the calculated cardiac R2* (1/T2*) in patients at risk for cardiac siderosis. The study group consisted of 36 patients with thalassemia syndromes who had undergone clinical magnetic resonance imaging assessment of cardiac siderosis using a standardized protocol and who were selected to yield a broad range of cardiac R2* values.

WO 2006/134430 A2 discloses an apparatus for analyzing the distribution of iron content in determined anatomic regions, in particular in the heart, of thalassemic patients subject to many blood transfusion and for which martial accumulation should be monitored. The apparatus provides magnetic resonance means adapted to measure a succession of images, for example at the left ventricle of the myocardium, for different echo times. By of suitable contour tracking means the image is defined so that the contours of the more relevant parts is highlighted.

ZHANG YAONAN et al.: "The shape analysis of ventricular septum based on medical images", 2013 IEEE INTERNATIONAL CONFERENCE ON MEDICAL IMAGING PHYSICS AND ENGINEERING, IEEE, 19 October 2013, pages 201-206 discloses a method in which, in order to make a diagnosis of cardiovascular disease, the edge of ventricular endometrial and skeleton line of ventricular septum are extracted by using computer-aided diagnosis techniques, and some shape analyses are done with the results, including the calculation of length of ventricular endometrial edge which plays an important role in analyzing heart contractions motion and the ventricular interval length which can be used as a reference when making a diagnosis of myocardial hypertrophy. An algorithm of auto-calculation of ventricular septum skeleton curvature is disclosed as well as the conception of ventricular septum offset. Calculation and data analysis of ventricular septum offset are presented. The evaluation of ventricular septum is achieved.

US 2004/153128 A1 discloses a computerized method of facilitating cardiac intervention. The method may include inputting patient data and creating a computerized interactive model of a heart based on the patient data. The model may include features. The model may simulate at least one proposed cardiac intervention by adding or deleting features to the model, and determining the effects of the proposed cardiac simulation upon the entire model. Simulations may be repeated to allow the user to determine an optimal cardiac intervention. A template and/or patient specific instrument may be created from the model to use as a guide during the cardiac intervention.

It is an object of the invention to provide methods and apparatus that enable MIC to be obtained more easily and/or more reliably.

According to an aspect of the present invention, there is provided a computer-implemented method of detecting myocardial iron content using black-blood magnetic resonance imaging data according to claim 1.

The inventors have found that the combination of the above contour fitting and the binary map-based segmentation provides a computationally efficient and reproducible way of identifying a set of voxels in the imaging data that belong to the interventricular septum. Using this automatically derived set of voxels to detect the myocardial iron content greatly facilitates the detection of the myocardial iron content in comparison with manual methods, because no manual work is required by the clinician. Furthermore, removing the manual interaction reduces the risk of intra- or inter-operator variability, leading to more reproducible and reliable results.

Fitting of circles to imaging data can be performed quickly and relatively easily. The inventors have also found that initialization of the contour fitting using such circles is computationally efficient and leads to reliable and rapid identification of the correct contours.

In an embodiment, the imaging data is processed to obtain an edge map and the fitting of the two circles to the imaging data comprise fitting of the two circles to the edge map, wherein the edge map is obtained using a spatially-constrained fuzzy c-means. The inventors have found that using spatially-constrained fuzzy c-means greatly increases the reliability with which the two different circles can be detected. In an embodiment, the adjustment step utilizes the Chan-Vese model. The inventors have found that this model is particularly effective for obtaining the contours starting from the fitted circles. Preferably, an improved version of the Chan-Vese model is used, as described in Zheng Q, Lu Z, Zhang M, Feng Q, Chen W. Gaussian Regularizing CV Model Using Entropy and Neighborhood Information. In: Proceedings of IFMBE, 2012, Beijing, China;New York: Springer; 2013. p. 1832-1835. This improved version of the Chan-Vese model is referred to herein as "I-CV".

Further embodiments of the invention will now be described, by way of example only, with reference to the accompanying drawings in which corresponding reference symbols indicate corresponding parts, and in which:

Figure 1 depicts a framework for a method according to an embodiment;
Figure 2 depicts a typical black-blood myocardial image;
Figure 3 depicts an edge map derived from the image of Figure 2;
Figure 4 depicts application of the circular Hough transformation (CHT) for detecting circular features in an image;
Figure 5 depicts a signature curve degenerated by applying the CHT to the edge map of Figure 3;
Figure 6 depicts two concentric circles identified by applying the CHT to the edge map of Figure 3;
Figure 7 depicts an example signature curve in a case where only one local minimum is present;
Figure 8 depicts the edge map used to generate the signature curve of Figure 7;
Figure 9 depicts the single circle identified by applying the CHT to the edge map of Figure 8;
Figure 10 depicts the signature curve obtained by applying the CHT to an improved edge map;
Figure 11 depicts the improved edge map used for obtained the signature curve of Figure 10;
Figure 12 depicts the two concentric circles derived from the signature curve of Figure 10;
Figure 13 depicts the results of applying I-CV segmentation;
Figure 14 depicts the fitting of ellipses to the results of Figure 13;
Figure 15 depicts the segmentation of myocardium
Figure 16 depicts a typical myocardial image;
Figure 17 depicts a binary map derived from magnetic resonance data based on thresholding;
Figure 18 depicts identification of angular boundaries of the interventricular septum;
Figure 19 depicts extraction of the interventricular septum based on the boundaries identified in Figure 18;
Figure 20 depicts a scatter plot of T2* measurements based on an interventricular septum segmented according to an embodiment ("AISS") and an interventricular septum segmented manually;
Figure 21 depicts a Bland-Altman plot of T2* measurements based on an interventricular septum segmented using AISS and an interventricular septum segmented manually;
Figure 22 depicts a Bland-Altman plot of intra-observer reproducibility for the manual method;
Figure 23 depicts a Bland-Altman plot of inter-observer reproducibility for the manual method;
Figure 24 depicts a Bland-Altman plot of intra-observer reproducibility for the AISS method;
Figure 25 depicts a Bland-Altman plot of inter-observer reproducibility for the AISS method;
Figure 26 depicts a Bland-Altman plot of agreement between the manual method and the "sAISS" method, which allows manual adjustment to outputs from the AISS method;
Figure 27 depicts a Bland-Altman plot of agreement between the AISS and sAISS methods; and
Figure 28 depicts an MRI machine and data analysis unit according to an embodiment.

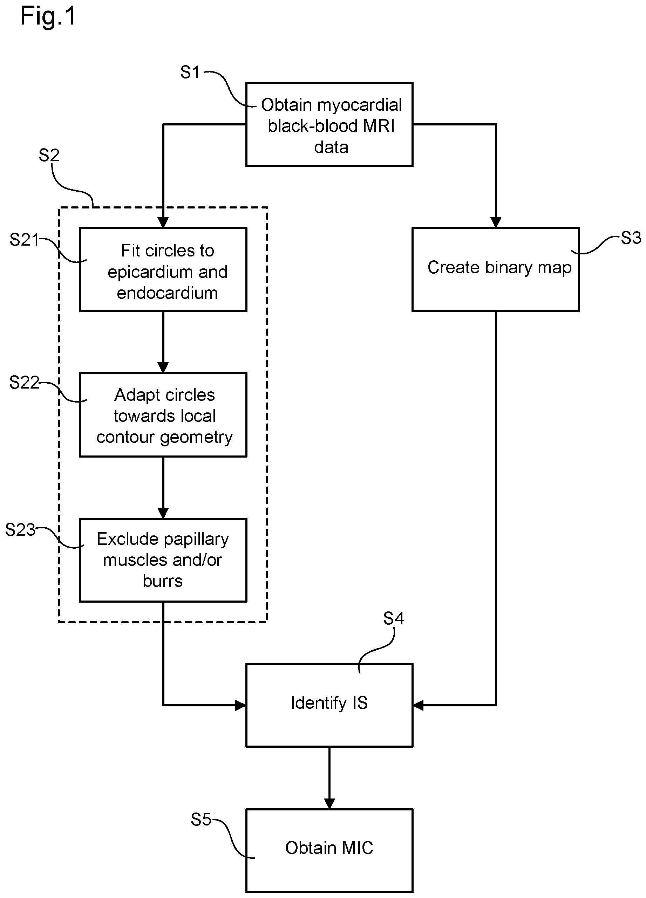

A framework for an example method is shown in Figure 1.

In step S1, MRI data of the patient is obtained. In an embodiment the MRI data is myocardial black-blood imaging data.

In step S2, contours are fitted to epicardial and endocardial margins of the left ventricle (LV) in the image data.

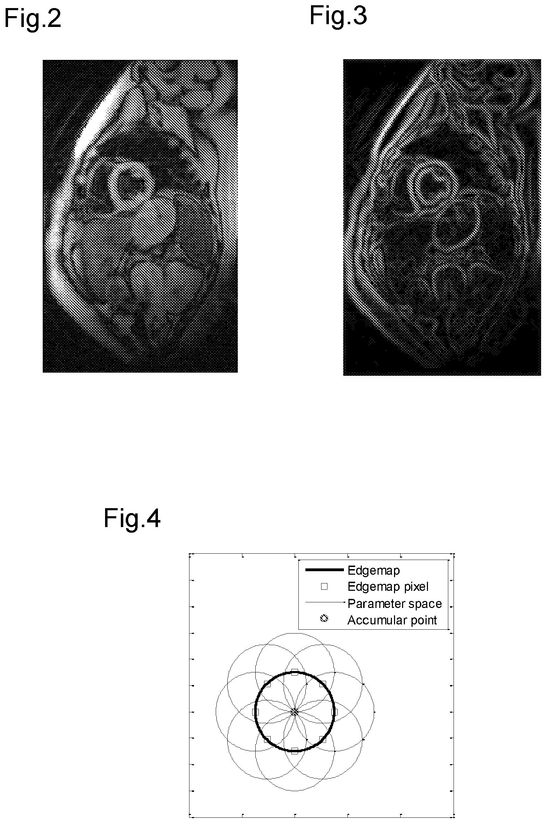

In an embodiment, step S2 comprises an initialization step S21 in which two circles are fitted to the image data, a first of the circles being fitted to the epicardial margin of the left ventricle and a second of the circles being fitted to the endocardial margin of the left ventricle. In an embodiment, the circles are fitted using the circular Hough transformation (CHT) (23). CHT is a widely-used method to detect circular structures in an image. Figures 2-6 illustrate the basic principle of detecting circles with a fixed radius in the CHT and show an example of its successful application to typical imaging data.

Figure 2 shows example black-blood mycocardial image data. This imaging data is processed to produce an edge map, as shown in Figure 3. The edge map may be derived by calculating the gradient of the imaging data and may be denoted as $EdgeM(x,y)$. Figure 4 illustrates how the CHT is applied. For each pixel in an edge map, a fixed circle centred at this pixel is plotted, and the points traced out by the circle produce a set of votes in the parameter space After accumulating the votes in an accumulator array for all pixels on the edge map, the array element with the highest number of votes indicates the centre of the circle. Because of the unknown radii of epicardial and endocardial contours, in the present example CHTs were implemented with radii varying from 8 pixels to 30 pixels with a 1-pixel increment, roughly corresponding to the typical size range of human hearts. The results of these CHTs were summed to an accumulator array with the centres of circles being determined by the peak points in the array. In the present example, a single peak point (or localised region) representing the centre of concentric circles lying roughly over the contours of the epicardium and endocardium is expected. Once the centre has been located, the radii of the two circles can be determined from the signature curve generated by histogramming the edge-weighted distances between the points on the edge map and the detected centre:

$$R(x,y) = EdgeM(x,y)\sqrt{(x-x_c)^2+(y-y_c)^2} \qquad [1]$$

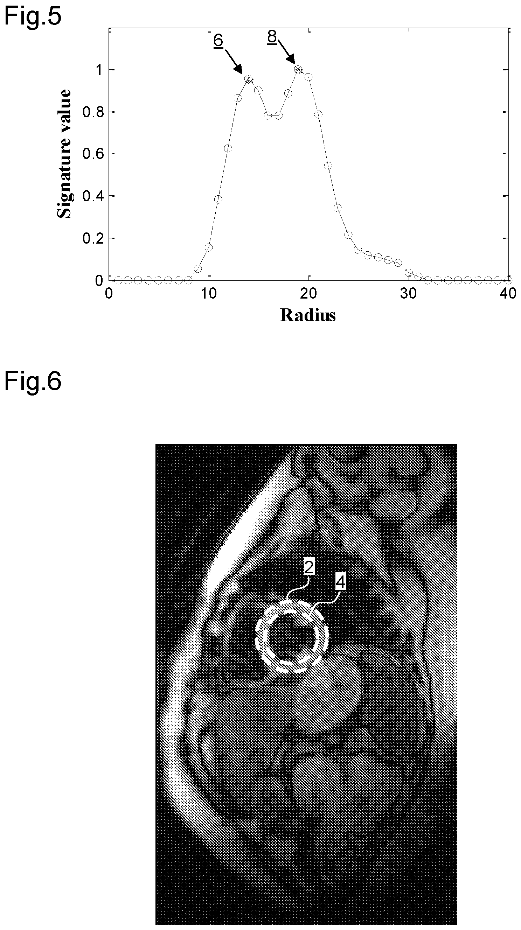

where $R(x,y)$ denotes the edge-weighted distance between the pixel $(x,y)$ and the centre $(x_c, y_c)$. Figure 5 depicts the generated signature curve. The local maxima 6 and 8 in the signature curve indicate the size of radii of the circles of interest. Figure 6 shows the two derived concentric circles 2 and 4 superimposed over the CMR image of Figure 2. It can be seen that the two circles 2,4 lie respectively over the contours of the epicardium and endocardium.

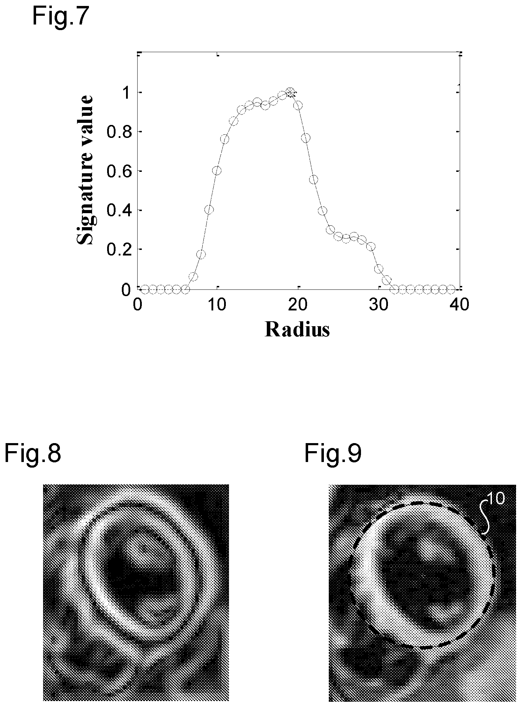

The performance of CHT depends on the quality of the edge map derived from an image. In the traditional CHT (23), the edge map is calculated as the gradient of image and hence is often affected by noise, partial volume effect (PVE), artifacts, etc. As a result, it is sometimes the case that only one local maximum exists in the signature curve, in which case only one circle will be detected. An example of a signature curve generated in such a situation is shown in Figure 7. In this case, the CHT was applied to an edge map shown in Figure 8 (obtained by calculating the gradient of the image). The resulting (single) circle 10, detected by the CHT, is plotted in Figure 9.

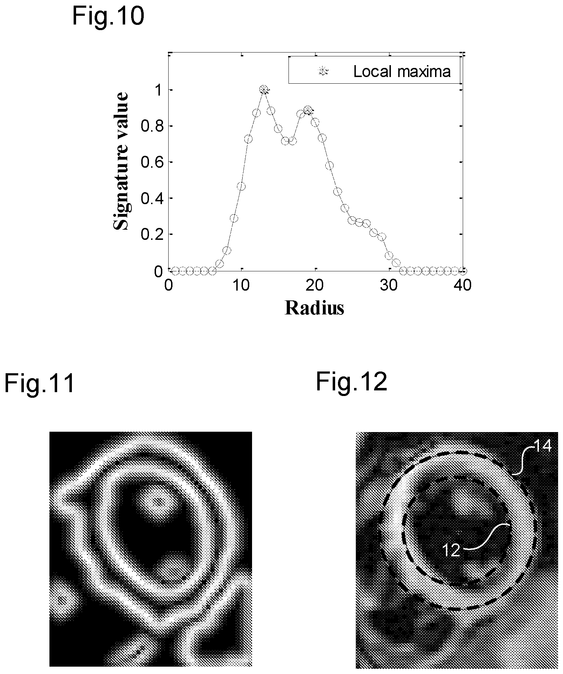

In an embodiment, this problem is addressed by binarizing the local image around the LV to obtain an improved edge map. In an embodiment this is achieved using spatially-constrained fuzzy c-means (sFCM) (24) with two clusters. Figure 10 shows the signature curve obtained by applying CHT to the improved edge map (shown in Figure 11). As can be seen, the improved edge map contains only the prominent edges and the resulting signature curve contains two clear local maxima. Figure 12 shows the two corresponding concentric circles 12 and 14 plotted over the CMR data.

In an embodiment, step S2 further comprises an adjustment step S22, in which the circles obtained in step S21 are iteratively adjusted towards the local contour geometry of the epicardium and endocardium. In an embodiment, the Chan-Vese (CV) model (25) is used for this purpose. The CV model has the ability to detect weak boundaries and is a widely used active contour model in image segmentation (26-29). However, the traditional Chan-Vese model assumes that the interior and exterior of the curve are statistically homogeneous, and this assumption does not hold in the entire myocardial T2* images (30), which generally contain complex structures and are affected by intensity inhomogeneity and flow artifacts. However, myocardial T2* images can be considered approximately homogeneous in small local regions, and this local characteristic can be exploited by using an improved Chan-Vese model, referred to here as "I-CV" with entropy-weighted and local neighborhood information to extract the LV from myocardial T2* images.

Let $I(z)$ denote an image defined on domain $\Omega$, and $C$ a closed curve which divides the image into object and background. The object is denoted by $\Omega_{IN}$, the interior of the curve, and the background is denoted by $\Omega_{EX}$, the exterior of the curve. The energy functional of I-CV model can be formulated as

$$\varepsilon(\phi) = \mu\int_{\Omega}\delta(\phi)|\nabla\phi|dz + \nu\int_{\Omega}H(\phi)dz + \int_{\Omega}\delta(\phi)\int_{x\in N_j}F_j(\phi)dxdz \qquad [2]$$

Here, $\phi$ denotes the level set function (31) where $C = \{z|\phi(z)=0\}$, $\phi(\Omega_{IN})> 0$ and $\phi(\Omega_{EX})<0$. The first term of the above energy functional represents the length of curve $C$, where $\delta(z)$ is the Dirac function and $\nabla\phi$ is the gradient of the level set function $\phi$. The second term represents the area enclosed by curve $C$, where $H(z)$ denotes the Heaviside function. The parameters $\mu$ and $\nu$ are nonnegative controlling parameters. The last term is called the data term derived from the image which takes on its smaller values at the features of interest such as boundaries, where $F_j$ denotes the fitting energy of a neighborhood centred at the pixel $j$ on the curve $C$. Let $N_j$ denote the neighborhood centreed at the point $j$ on the closed curve $C$, the calculation of $F_j$ can be formulated as

$$F_j(\phi) = E_{in}^j(\phi)\left|I(x)-u_j\right|^2 H(\phi) + E_{ex}^j(\phi)\left|I(x)-v_j\right|^2(1-H(\phi)) \qquad [3]$$

where $E_{in}^j$ and $E_{ex}^j$ denote the entropies of the interior and exterior of the neighborhood $N_j$, separated by the curve $C$; $u_j$ and $v_j$ represent the average intensities of the interior and exterior of the neighborhood $N_j$, respectively. The entropies $E_{in}^j$ and $E_{ex}^j$ can be calculated as

$$E_{in}^j(\phi) = -\int_{x\in N_j} H(\phi(x))p(I(x))\log p(I(x))dx \qquad [4]$$

$$E_{ex}^j(\phi) = -\int_{x\in N_j}(1-H(\phi(x)))p(I(x))\log p(I(x))dx \qquad [5]$$

where $p(I(x))$ denotes the probability density function of intensities in the whole image. The mean intensities $u_j$ and $v_j$ are calculated as

$$u_j = \frac{\int_{x\in N_j} I(x)H(\phi(x))dx}{\int_{x\in N_j} H(\phi(x))dx}, \quad v_j = \frac{\int_{x\in N_j} I(x)(1-H(\phi(x)))dx}{\int_{x\in N_j}(1-H(\phi(x)))dx} \qquad [6]$$

The length term and area terms in the I-CV model regularize that the contour is smooth during the evolution. The data term moves the curve C to target edges using the information from the image.

Minimizing the above energy function by using the steepest descent method (32), we obtain the following variational formulation

$$\frac{\partial\phi(z)}{\partial t} = \delta(\phi(z))\left[\mu div\left(\frac{\nabla\phi}{|\nabla\phi|}\right) - \nu - \int_\Omega \nabla_\phi F_j(\phi)dx\right] \qquad [7]$$

where $\nabla_\phi F_j(\phi) = \delta(\phi(x))(E_{in}^j(\phi)\left|I(x)-u_j\right|^2 - E_{ex}^j(\phi)\left|I(x)-v_j\right|^2)$.

In an embodiment, the epicardial and endocardial contours are automatically segmented (i.e. identified) by using the iteration depicted in Eq. [7]. However, the I-CV extracted LV usually contains the papillary muscles and the extracted contours may have sharp burrs. In addition, the pixels on the extracted contours are likely affected by Partial Volume Effects (PVE) which should be excluded from T2* measurement.

In an embodiment, step S2 further comprises a processing step S23 for reducing or excluding contributions from the papillary muscles and/or burrs.

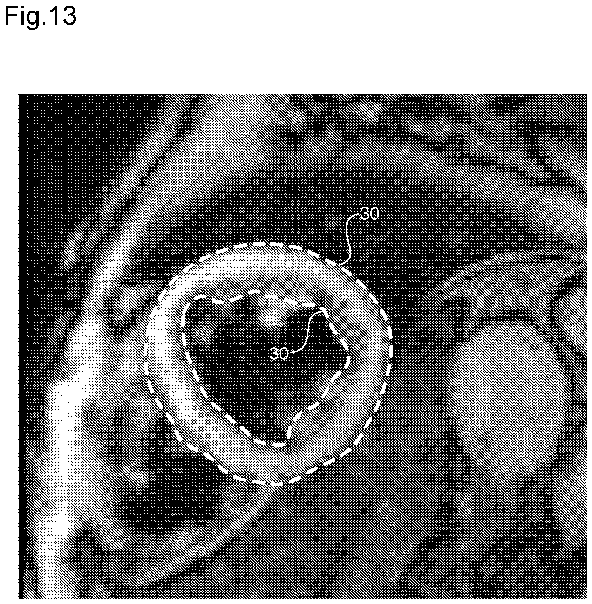

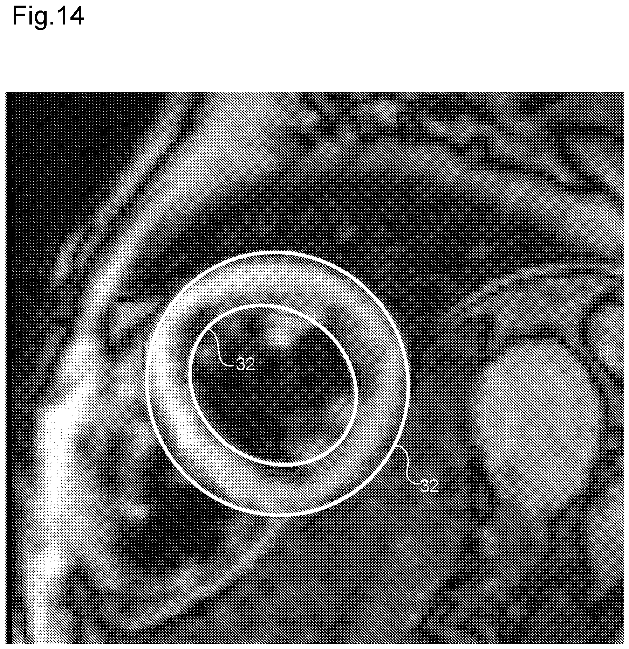

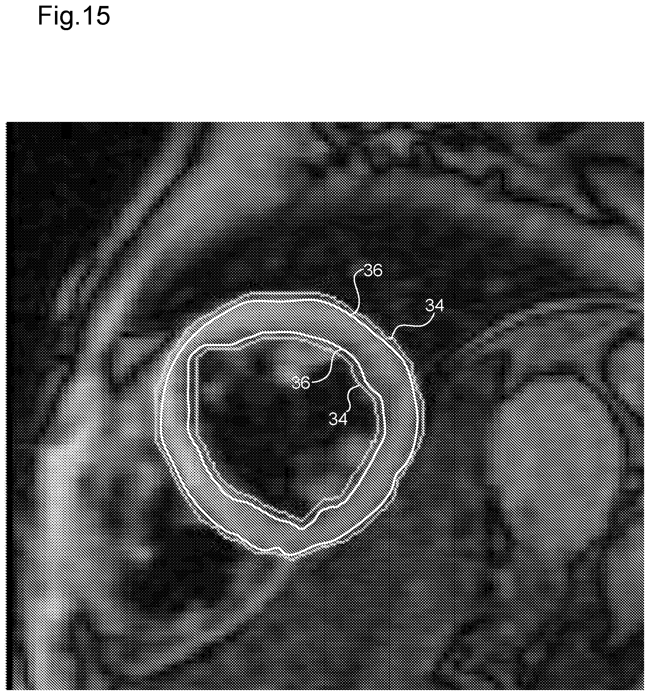

The following describes an embodiment for excluding, or at least reducing the errors in the estimated contours caused by, the papillary muscles and burrs in step S23. In this context, the above-described method of step S22 of iteratively adjusting the fitted circles (obtained in step S21) may be referred to as a "first adjustment step". The estimated contours of the left ventricle thus obtained may be referred to as the "first estimated contours". Examples of such first estimated contours 30 are depicted in Figure 13. The two first estimated contours 30 will often have significant burrs (irregularities arising from the fitting which do not represent the real morphology of the biological structure) and be significantly affected by the presence in the image of the papillary muscles. In the present embodiment a second adjustment step is then performed, as part of step S23. The second adjustment step comprises fitting an ellipse to each of the first estimated contours obtained in the first adjustment step S22, thereby obtaining first estimated ellipses. Examples of such first estimated ellipses 32 are depicted in Figure 14. A third adjustment step is then performed, as a further part of step 23. The third adjustment step comprises obtaining second estimated contours. The second estimated contours are determined by identifying the margins of the portion of the myocardium that is enclosed by both of first estimated contours and the first estimated ellipses. This step is particularly effective at reducing the influence of the papillary muscles. Examples of such second estimated contours 34 are depicted in Figure 15. In an embodiment, a fourth adjustment step is then performed, as a still further part of the step 23. The fourth adjustment step comprises moving an outermost second estimated contour 34 inwards and an innermost second estimated contour 34 outwards by a predetermined distance or predetermined number of pixels. In an embodiment, the predetermined number is one pixel. This step tends to exclude pixels likely to be affected by the partial volume effect, thereby improving accuracy. The overall process significantly improves the accuracy of the final estimated contours, excluding, or at least reducing the errors in the estimated contours caused by, the papillary muscles, burrs, and/or partial volume effects.

In a further step S3, a binary map is created by segmenting voxels of the image data according to whether the voxel values are above or below a threshold. The binary map can be used to determine the portion (e.g. angular range) of the epicardial and endocardial contours obtained in step S2 that corresponds to the interventricular septum (IS). The IS thus identified can then be used to obtain the myocardial iron content.

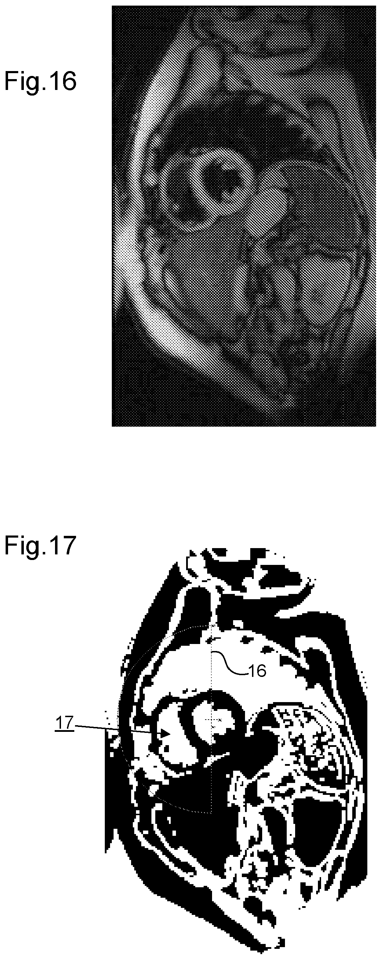

In an embodiment the threshold is determined automatically based on the blood pool in the LV. A threshold is chosen that distinguishes clearly between the blood and the tissue of the LV. In black-blood T2* images, the intensities of the blood pool are far lower than those of the myocardium. This characteristic ensures that threshold-based segmentation is a suitable method for extracting the RV. In an embodiment, the threshold value is determined by calculating the mean and standard deviation (SD) of the intensities in the blood pool of the LV, as delimited by the contours extracted in step S2, and setting the threshold equal to the mean+SD. Figure 16 shows a typical myocardial image and Figure 17 shows the binary result of thresholding.

In an embodiment, the binary map created in step S3 is used to identify the blood pool of the RV. In an embodiment, the blood pool of the RV is identified as the largest, closed blood feature within a predetermined local region on the right of the LV. In the example shown in Figure 17, the local region is the half disc area 16. The blood pool of the RV is indicated by 17.

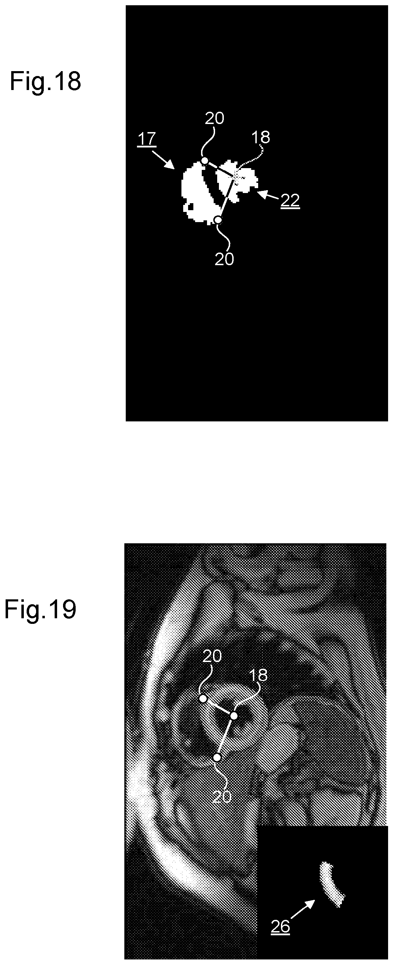

In step S4, the fitted contours obtained in step S2 and the binary map obtained in step S3 are used to identify the portion of the tissue between the fitted contours corresponding to the IS. In an embodiment, the binary map is used to identify the upper and lower margins of the IS. In an embodiment, this is achieved by determining the angles at which radial lines originating from the centre of LV 22 intersect the region 17 of the extracted blood pool of RV at only one point, as shown in Figure 18. The centre of the LV 22 is marked 18 and the single points of intersection with the region 17 of the extracted blood pool of RV are marked 20. Combined with epicardial and endocardial contours obtained from step S2, the IS can be extracted as shown in Figure 19. The resulting IS 26 is shown in the bottom right inset.

In an embodiment, the extraction of IS as described above is performed in a fully automatic way (i.e. entirely by a computer). However, this is not essential. In other embodiments, it is possible to provide a user with the option to adjust the results of one or more of the fitting steps. For example, provision may be made to allow a user manually to adjust the contours of the IS obtained in step S4. Alternatively or additionally, provision may be made to allow a user manually to adjust the results of any of the fitting processes used in the overall method, including any or all of the steps S21, S22, S23, S3 and S4. In an embodiment, provision may be made to allow a user to adjust the upper and lower margins of the IS, for example by manually adjusting the positions of one or both of the points 20 described above with reference to Figures 18 and 19 and/or the angle between the radial lines extending from the centre of LV 22.

In step S5, the mycardial iron content is detected by performing T2* measurements on the portion of the imaging data corresponding to the identified IS. In an embodiment, the signal intensities in the IS are measured for each of the images at multiple TEs, and the IS-averaged data are fitted to an exponential decay model to derive the T2* value: $S(TE) = S_0 \cdot e^{-TE/T2^*}$, where $S(TE)$ denotes the signal intensity at TE and $S_0$ represents the signal intensity at zero TE. No truncation is needed for the black-blood myocardial T2* measurements as previously reported (18). In an embodiment, the fitting is performed using the Levenberg-Marquardt nonlinear optimization method (19) with positive constraints of $S_0$ and T2*.

The inventors performed detailed statistical analyses to demonstrate that the automated method works effectively in the context of real data. In the following, fully automated methods involving no user input are referred to as "AISS" methods (which stands for "Automated Interventricular Septum Segmentation"). Methods involved a degree of user input in addition to the automatic steps (for example to manual adjust contours, etc.) are referred to as "sAISS" methods (standing for "semi-Automated Interventricular Septum Segmentation").

T2* measurements were obtained in a total of 144 thalassemia patients (73 males and 71 females, mean age 27 years) with varying degrees of MIC using a 1.5T scanner (Siemens Sonata, Erlangen, Germany) with a four-element cardiac phased-array coil and standard electrocardiogram gating. A single short-axis midventricular slice positioned halfway between the base and the apex of the left ventricular (LV) was imaged by using a black-blood, multi-echo, gradient-echo sequence (flip angle 20°, sampling bandwidth 810 Hz/pixel, voxel size $1.56\times1.56\times10$ mm$^3$ and variable field of view $(20\sim30)\times40$ cm$^2$ depending on patient size). The short-axis images were acquired at 8 echo times (TE) from 2.54 ms to 17.90 ms with an increment of approximately 2 ms in a single breath-hold. The repetition time between radiofrequency pulses was 20 ms.

Reproducibility was assessed by using the mean and SD of the differences as described by the Bland-Altman analysis (20). The level of agreement between two methods was represented by the interval of the percentage difference between mean$\pm1.96$SD. In addition, for accessing the intra- and inter-observer variability, the coefficient of variation (CoV) was defined as the SD of the differences between the two independent measurements divided by their means and presented as a percentage. Correlation analysis was performed using Pearson's test. For all statistical analysis, a P value of < 0.05 was considered to be statistically significant.

In the current study, methods according to the above described embodiments were performed on a PC using in-house developed software (MATLAB 2012a; The MathWorks, Natick, MA). T2* measurments using manual segmentation outlined by two trained observers were considered as reference. The data were randomized and anonymized before the T2* measurement, and the two observers were blinded to each other's results.

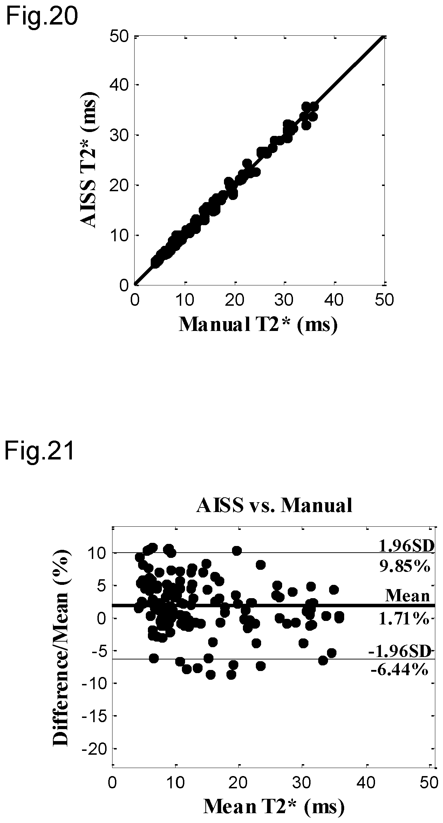

Figures 20 and 21 show the scatter and Bland-Altman plots of T2* measurements from an AISS method and manual methods applied to 144 transfusion-dependent patients. The difference had a mean of 1.71%, and a confidence interval of (-6.44%, 9.85%). The CoV between T2* measurments using the AISS and manual methods was 4.15% (r=0.9978, P<0.001).

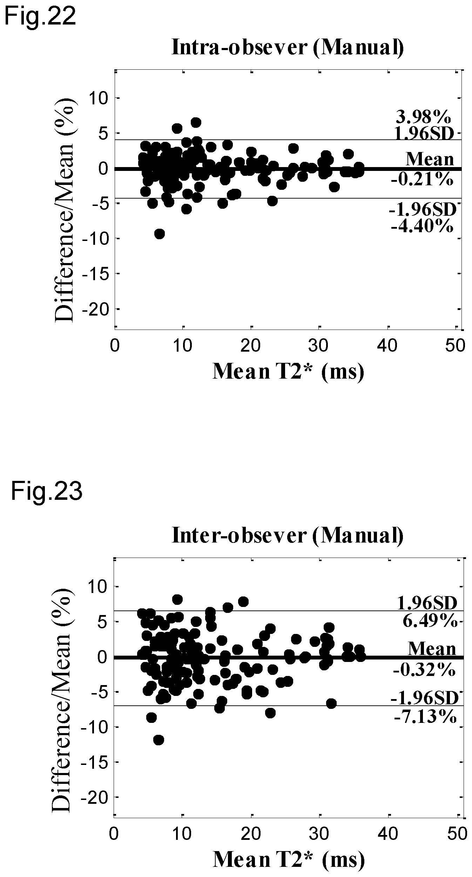

Figures 22 and 23 show the Bland-Altman plots of the intra- and inter-observer reproducibility for the manual method. The intra-observer differences for the manual method had a mean of -0.21% and an interval of (-4.40%, 3.98%), and the inter-observer differences had a mean of -0.32% and an interval of (-7.13%, 6.49%). The CoVs of the intra- and inter-observer variabilities were 1.90% (r=0.9995, P<0.001) and 3.37% (r=0.9986, P<0.001), respectively.

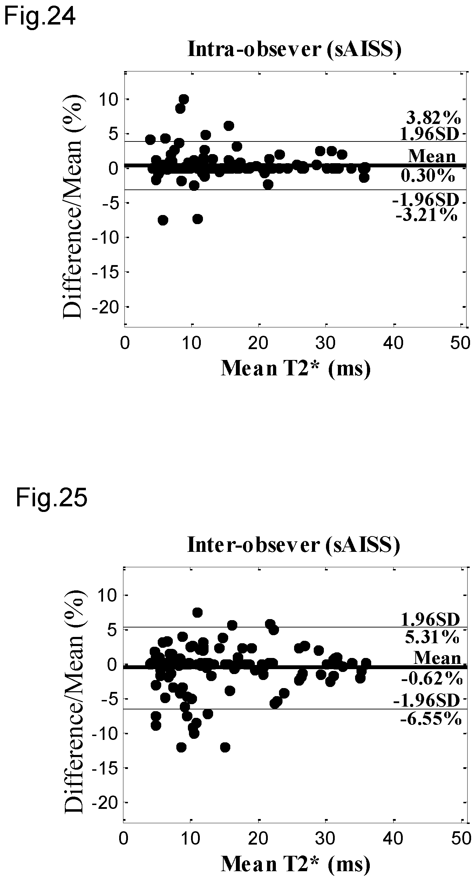

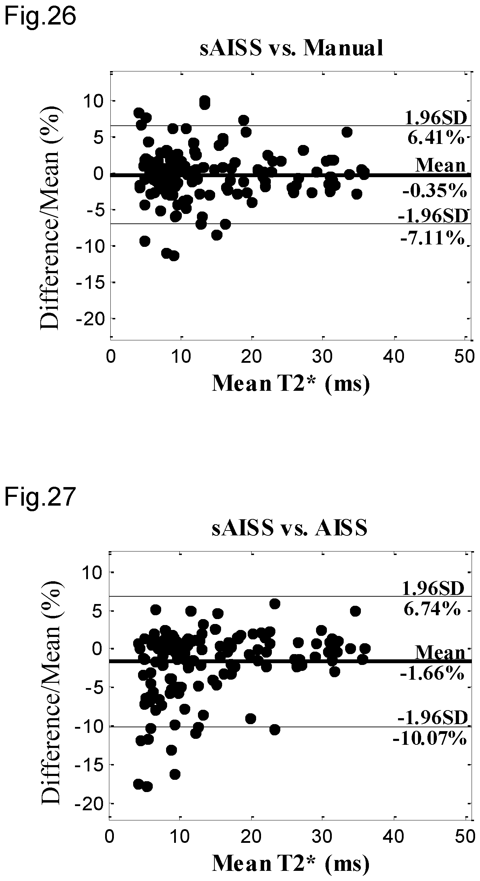

For a semi-automated method, sAISS, where a user is able to manually adjust the results of the automatic fittings, Figures 24 and 25 present the Bland-Altman plots of the intra- and inter-observer reproducibilities, respectively. The intra-observer difference for the sAISS method had a mean of 0.30% and an interval of (-3.21%, 3.82%), and the inter-observer difference for the sAISS method had a mean of 0.30% and an interval of (-3.21%, 3.82%). The CoVs of the intra- and inter-observer reproducibilities for the sAISS method were separately 1.38% (r=0.9998, P<0.001) and 2.71% (r =0.9991, P<0.001), either of which was lower than that of the manual method. Figures 26 and 27 show the Bland-Altman plots of the agreements between the sAISS and manual methods, and between the sAISS and AISS methods. The small difference intervals and low CoV values indicate that sAISS has a good agreement with both the manual and AISS method. The CoV between sAISS and manual was 3.08% (r=0.9988, P<0.001), and the CoV between sAISS and AISS was 3.27% (r=0.9987, P<0.001), both of which were slightly lower than that between AISS and manual (4.15%).

In the present example, the I-CV model was used for the extraction of LV margins/contours. This model introduces the local neighborhood information to reduce the effect of intensity inhomogeneity on the evolving curves (15). Similar to other active contour models, the I-CV model requires an initialization, which was usually manually determined. In the present example, the CHT was used to automatically detect the concentric circles in cardiac images as an initialization. With direct application of CHT on the original T2* images, the endocardium and epicardium were successfully detected in 86.1% of the 144 datasets, and only one circle was initialized near the myocardial area in the rest of the datasets (13.9%). For the latter scenario, the sFCM (9) was implemented in a local area surrounding the detected circle to binarize the image for the purpose of providing a better edge map for the CHT, and the result demonstrated that the combination of sFCM and CHT successfully detected two concentric circles as a good initialization approach.

The susceptibility was lower in IS than other regions of the LV myocardium, and T2* values calculated from IS provided lower intra- and inter-observer variabilities (6). Therefore, the IS T2* measurement is widely reported to reflect MIC in clinical practice (4, 5, 7). Thalassaemia Tool (a plug-in of CMR tools, Cardiovascular imaging Solutions, London, UK), a widely used myocardial T2* analysis software, uses two radial lines from the LV centre to the LV/RV junction points for segmenting the septal region (5). The IS was extracted in a similar but automated manner in the proposed AISS method. Specifically, the two radial lines starting from the LV centre in the AISS method were automatically determined as the ones with the smallest separation angle among those enclosing the blood pool of RV.

In the present example, the AISS method was fully automated, and took approximately 1.4 s to fulfill the T2* analysis of one dataset. The Bland-Altman plot and CoV between the AISS and manual methods demonstrates that the AISS method is in good agreement with the manual method. The population mean T2* of the AISS-defined IS was slightly higher than that of manual method (mean difference, 1.7%), which is probably because the IS extracted by AISS is generally larger than that extracted by the manual method, which confines T2* measurement in a narrow IS to exclude high T2* pixels near the myocardial edges (7).

The provision of a semi-automated method, "sAISS" method provides a computer-aided operation for clinicians to further adjust the AISS-extracted IS with ease. In the implementation of the sAISS method, approximately 45% of the AISS-extracted IS were further refined by the observers on average in this patient group. Compared with the manual method, the CoVs of the intra- and inter-observer reproducibilities for sAISS method were both decreased. The narrowed intervals of difference and decreased values of CoV using the sAISS method indicate that this method has improved intra- and inter-observer reproducibilities compared with the manual method, owing to the reduced subjectivity. As would be expected, the Bland-Altman plots among the sAISS, AISS and manual methods demonstrated that sAISS agrees better with the manual method than AISS due to the intervention from observers (mean difference, -0.35% vs. 1.71%).

Embodiments of the invention are designed specifically for the black-blood T2* technique, which can largely reduce blood signal contamination from the myocardium and thus provides superior myocardial border definition than the bright-blood T2* technique (5). Bright-blood T2* relaxometry is more widely available but has intensity characteristic distinct from the black-blood images, due to flow artifacts and poorer contrast

Except where explicitly stated to the contrary, any one or all of the steps of the methods described above can be performed using a suitably programmed computer comprising hardware such as CPU and RAM with which the skilled person would be very familiar.

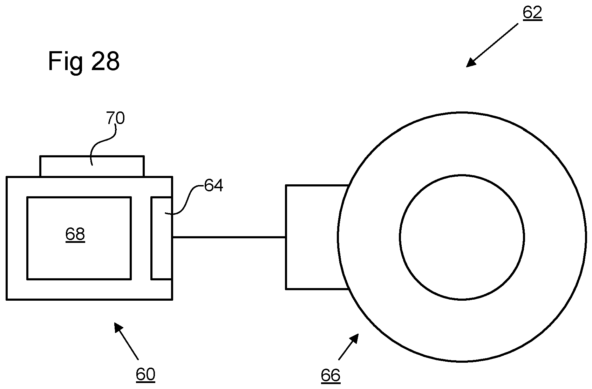

As shown in Figure 28, in an embodiment, a data analysis unit 60 is provided that may optionally form part of an MRI machine 62 (or be separate from it). The data analysis unit 60 may comprise an input unit 64 for receiving MRI data, directly or indirectly, from an MRI data acquisition system 66. The data analysis unit 60 may further comprise a data processing unit 68 (comprising standard computer hardware for example) that is configured to carry out any one or all of the steps of the methods described above. A computer program may be provided for example (e.g. via a computer medium storing the computer program or via a network or other data connection) that comprises instructions which, when run on a computer, for example the data processing unit 68, cause the computer to carry out any one or all of the steps of the method described above.

In an embodiment, the data analysis unit 60 further comprises a user interface 70 for allowing a user to manually interact with the data analysis unit 60, wherein the data analysis unit 60 is configured to allow a user to adjust the results of one or more of the steps of fitting contours to the epicardial and endocardial margins of the left ventricle, creating the binary map, and using the created binary map to identify the interventricular septum, using the user interface 70. For example, the user may use the user interface 70 to move pixels associated with the fitted contours of the epicardial or endocardial margins, to adjust the angle of one or both of the radial lines extending from the centre of the left ventricular blood pool, or move pixels defining the boundary of the interventricular septum (as obtained by the automated methods discussed above).

References

1. Pennell DJ. T2* magnetic resonance: iron and gold. JACC Cardiovasc Imaging 2008;1(5):579-581.

2. Tanner MA, He T, Westwood MA, Firmin DN, Pennell DJ. Multi-center validation of the transferability of the magnetic resonance T2* technique for the quantification of tissue iron. Haematologica 2006;91(10):1388-1391.

3. Daar S, Pathare AV, Jain R, Zadjali SA, Pennell DJ. T2* cardiovascular magnetic resonance in the management of thalassemia patients in Oman. Haematologica 2009;94(1):140-141.

4. Smith GC, Carpenter JP, He T, Alam MH, Firmin DN, Pennell DJ. Value of black blood T2* cardiovascular magnetic resonance. J Cardiovasc Magn Reson 2011;13:21.

5. He T, Gatehouse PD, Kirk P, et al. Black-blood T2* technique for myocardial iron measurement in thalassemia. J Magn Reson Imaging 2007;25(6):1205-1209.

6. Yamamura J, Grosse R, Graessner J, Janka GE, Adam G, Fischer R. Distribution of cardiac iron measured by magnetic resonance imaging (MRI)-R*2. J Magn Reson Imaging 2010;32(5):1104-1109.

7. Saiviroonpom P, Viprakasit V, Boonyasirinant T, Khuhapinant A, Wood JC, Krittayaphong R. Comparison of the region-based and pixel-wise methods for cardiac T2* analysis in 50 transfusion-dependent Thai thalassemia patients. J Comput Assist Tomo 2011;35(3):375-381.

8. Illingworth J, Kittler J. The adaptive hough transform. IEEE Trans Pattern Anal Mach Intell 1987;9(5):690-698.

9. Chuang KS, Tzeng HL, Chen S, Wu J, Chen T. Fuzzy c-means clustering with spatial information for image segmentation. Comput Med Imag Grap 2006;30(1):9-15.

10. Chan TF, Vese LA. Active contours without edges. IEEE Trans Image Process 2001;10(2):266-277.

11. Jonasson L, Bresson X, Thiran JP, Wedeen VJ, Hagmann P. Representing diffusion MRI in 5-D simplifies regularization and segmentation of white matter tracts. IEEE Trans Med Imaging 2007;26(11):1547-1554.

12. Maska M, Danek O, Garasa S, Rouzaut A, Munoz-Barrutia A, Ortiz-de-Solorzano C. Segmentation and shape tracking of whole fluorescent cells based on the Chan-Vese model. IEEE Trans Med Imaging 2013;32(6):995-1006.

13. Abdoli M, Dierckx RA, Zaidi H. Contourlet-based active contour model for PET image segmentation. Med Phys 2013;40(8):082507.

14. Lee SH, Seo JK. Level set-based bimodal segmentation with stationary global minimum. IEEE Trans Image Process 2006;15(9):2843-2852.

15. Zheng Q, Lu Z, Zhang M, Feng Q, Chen W. Gaussian Regularizing CV Model Using Entropy and Neighborhood Information. In: Proceedings of IFMBE, 2012, Beijing, China;New York: Springer; 2013. p. 1832-1835.

16. Osher S, Sethian JA. Fronts propagating with curvature-dependent speed: algorithms based on Hamilton-Jacobi formulations. J Comput Phys 1988;79(1):12-49.

17. Aubert G, Kornprobst P. Mathematical problems in image processing: partial differential equations and the calculus of variations. New York: Springer; 2006.

18. He T, Zhang J, Carpenter JP, et al. Automated truncation method for myocardial T2* measurement in thalassemia. J Magn Reson Imaging 2013;37(2):479-483.

19. Marquardt DW. An algorithm for least-squares estimation of nonlinear parameters. J Soc Ind Appl Math 1963;11(2):431-441.

20. Martin Bland J, Altman D. Statistical methods for assessing agreement between two methods of clinical measurement. Lancet 1986;327(8476):307-310.

**Claims**

1. A computer-implemented method of detecting myocardial iron content using black-blood magnetic resonance imaging data, comprising:

    fitting contours (S2) to the epicardial and endocardial margins of the left ventricle in the imaging data;

    creating a binary map (S3) by segmenting voxels of the imaging data according to whether the voxel values are above or below a threshold, the threshold being chosen so as to distinguish between blood and ventricular tissue;

    using the created binary map and the fitted contours to identify (S4) the portion of the tissue between the fitted contours corresponding to the interventricular septum; and

    detecting myocardial iron content (S5) by performing T2* measurements on a portion of the imaging data corresponding to the identified interventricular septum,

    wherein the step of fitting contours comprises:

        an initialization step (S21) in which two circles are fitted to the imaging data, a first of the circles being fitted to the epicardial margin of the left ventricle and a second of the circles being fitted to the endocardial margin of the left ventricle; and

        an adjustment step (S22) in which the fitted circles are iteratively adjusted to fit the respective margins of the left ventricle,

    and wherein the adjustment step (S22) comprises the following steps:

        a first adjustment step comprising iteratively adjusting the fitted circles to obtain first estimated contours (30) of the left ventricle;

        a second adjustment step comprising fitting an ellipse to each of the first estimated contours (30) obtained in the first adjustment step, thereby obtaining first estimated ellipses (32); and

        a third adjustment step comprising obtaining second estimated contours (34) as the margins of the portion of the myocardium that is enclosed by both said first estimated contours (30) and said first estimated ellipses (32).

2. A method according to claim 1, wherein the imaging data is processed to obtain an edge map and the fitting of the two circles to the imaging data comprise fitting of the two circles to the edge map.

3. A method according to claim 1 or 2, wherein the fitting of the two circles uses the circular Hough transformation.

4. A method according to any of claims 1-3, wherein the adjustment step (S22) utilizes the Chan-Vese model.

5. A method according to any preceding claim, further comprising:

a fourth adjustment step comprising moving an outermost second estimated contour (34) inwards and an innermost second estimated contour (34) outwards by a predetermined distance or predetermined number of pixels.

6. A method according to claim 5, wherein the predetermined number of pixels is one pixel.

7. A method according to any of the preceding claims, wherein the step of using the created binary map (S4) to identify the interventricular septum comprises:

identifying the blood pool of the right ventricle from the binary map;
determining the two different angles at which a single intersection occurs between a radial line extending from the centre of the left ventricle (22) and the identified blood pool (17) of the right ventricle; and
identifying the interventricular septum as the region within the fitted contours identifying the epicardial and endocardial margins and the two radial lines extending at the determined two angles.

8. A method according to claim 7, wherein the identifying of the blood pool of the right ventricle comprises identifying the largest, closed blood feature within a predetermined region on the side of the left ventricle where the blood pool of the right ventricle is expected to be found.

9. A method according to any of the preceding claims, wherein the threshold is based on a mean of voxel values in the blood pool in the left ventricle, the blood pool being identified as the region within the contour fitted to the endocardial margin.

10. A data analysis unit (60) for detecting myocardial iron content using black-blood magnetic resonance imaging data, comprising:

an input unit (64) configured to receive the imaging data;
a data processing unit (68) configured to perform the following steps:

fitting contours (S2) to the epicardial and endocardial margins of the left ventricle;
creating a binary map (S3) by segmenting voxels of the imaging data according to whether the voxel values are above or below a threshold, the threshold being chosen so as to distinguish between blood and ventricular tissue;
using the created binary map to identify (S4) the portion of the tissue between the fitted contours corresponding to the interventricular septum; and
detecting myocardial iron content (S5) by performing T2* measurements on a portion of the imaging data corresponding to the identified interventricular septum,

wherein the step of fitting contours comprises:

an initialization step (S21) in which two circles are fitted to the imaging data, a first of the circles being fitted to the epicardial margin of the left ventricle and a second of the circles being fitted to the endocardial margin of the left ventricle; and
an adjustment step (S22) in which the fitted circles are iteratively adjusted to fit the respective margins of the left ventricle,

and wherein the adjustment step (S22) comprises the following steps:

a first adjustment step comprising iteratively adjusting the fitted circles to obtain first estimated contours (30) of the left ventricle;
a second adjustment step comprising fitting an ellipse to each of the first estimated contours (30) obtained in the first adjustment step, thereby obtaining first estimated ellipses (32); and
a third adjustment step comprising obtaining second estimated contours (34) as the margins of the portion of the myocardium that is enclosed by both said first estimated contours (30) and said first estimated ellipses (32).

11. A data analysis unit (60) according to claim 10, further comprising:
a user interface (70) allowing a user to manually interact with the data analysis unit (60), wherein the data analysis unit (60) is configured to allow a user to adjust the results of one or more of the steps of fitting contours to the epicardial and endocardial margins of the left ventricle, creating the binary map, and using the created binary map to identify the interventricular septum, using the user interface (70).

12. An MRI machine (62), comprising:

a data acquisition system (66) for acquiring black-blood magnetic resonance imaging data; and
the data analysis unit (60) of claim 10.

13. A computer program for detecting myocardial iron content using black-blood magnetic resonance imaging data, the computer program being such that when run on a computer it causes the computer to perform the method of claim 1.

**Patentansprüche**

1. Computerimplementiertes Verfahren zum Detektieren eines Herzmuskeleisengehalts unter Verwendung von Schwarzblut-Magnetresonanzbildgebungsdaten, das Folgendes umfasst:

Ansetzen von Konturen (S2) an die epikardiale und die endokardiale Begrenzung der linken Herzkammer in den Bildgebungsdaten;
Erstellen einer binären Karte (S3) durch Segmentieren von Voxeln der Bildgebungsdaten davon abhängig, ob die Voxelwerte über oder unter einem Schwellwert liegen, wobei der Schwellwert ausgewählt wird, um zwischen Blut und Herzkammergewebe zu unterscheiden;
Verwenden der erstellten binären Karte und der angepassten Konturen, um den Abschnitt des Gewebes zwischen den angepassten Konturen, der dem Kammerseptum entspricht, zu identifizieren (S4); und
Detektieren eines Herzmuskeleisengehalts (S5) durch Durchführen von T2*-Messungen an einem Abschnitt der Bildgebungsdaten, der dem identifizierten Kammerseptum entspricht,
wobei der Schritt des Ansetzens von Konturen Folgendes umfasst:

einen Initialisierungsschritt (S21), bei dem zwei Kreise an die Bildgebungsdaten angesetzt werden, wobei ein erster der Kreise an die epikardiale Begrenzung der linken Herzkammer angesetzt wird und ein zweiter der Kreise an die endokardiale Begrenzung der linken Herzkammer angesetzt wird; und
einen Anpassungsschritt (S22), bei dem die angesetzten Kreise iterativ angepasst werden, um an den jeweiligen Begrenzungen der linken Herzkammer anzusitzen,

und wobei der Anpassungsschritt (S22) die folgenden Schritte umfasst:

einen ersten Anpassungsschritt, der das iterative Anpassen der angesetzten Kreise umfasst, um erste geschätzte Konturen (30) der linken Herzkammer zu erhalten;
einen zweiten Anpassungsschritt, der das Ansetzen einer Ellipse an jede der ersten geschätzten Konturen (30), die im ersten Anpassungsschritt erhalten wurden, um dadurch erste geschätzte Ellipsen (32) zu erhalten, umfasst; und
einen dritten Anpassungsschritt, der das Erhalten von zweiten geschätzten Konturen (34) als die Begrenzungen des Abschnitts des Herzmuskels, der sowohl von den ersten geschätzten Konturen (30) als auch den ersten geschätzten Ellipsen (32) umschlossen ist, umfasst.

2. Verfahren nach Anspruch 1, wobei die Bildgebungsdaten verarbeitet werden, um eine Kantenkarte zu erhalten, und das Ansetzen der beiden Kreise an die Bildgebungsdaten das Ansetzen der beiden Kreise an die Kantenkarte umfasst.

3. Verfahren nach Anspruch 1 oder 2, wobei das Ansetzen der beiden Kreise die Hough-Transformation für Kreise verwendet.

4. Verfahren nach einem der Ansprüche 1-3, wobei der Anpassungsschritt (S22) das Chan-Vese-Modell nutzt.

5. Verfahren nach einem der vorhergehenden Ansprüche, das ferner Folgendes umfasst:
einen vierten Anpassungsschritt, der das Bewegen einer äußersten zweiten geschätzten Kontur (34) nach innen und einer innersten zweiten geschätzten Kontur (34) nach außen um eine vorbestimmte Distanz oder eine vorbestimmte Anzahl von Pixeln umfasst.

6. Verfahren nach Anspruch 5, wobei die vorbestimmte Anzahl von Pixeln ein Pixel ist.

7. Verfahren nach einem der vorhergehenden Ansprüche, wobei der Schritt des Verwendens der erstellten binären Karte (S4), um das Kammerseptum zu identifizieren, Folgendes umfasst:

Identifizieren des Blutpools der rechten Herzkammer anhand der binären Karte;
Bestimmen der zwei verschiedenen Winkel, in denen zwischen einer radialen Linie, die sich von der Mitte der linken Herzkammer (22) und dem identifizierten Blutpool (17) der rechten Herzkammer erstreckt, ein einzelner Schnittpunkt erfolgt; und
Identifizieren des Kammerseptums als die Region innerhalb der angesetzten Konturen, die die epikardiale und die endokardiale Begrenzung identifizieren und der zwei radialen Linien, die sich in den bestimmten zwei Winkeln erstrecken.

8. Verfahren nach Anspruch 7, wobei das Identifizieren des Blutpools der rechten Herzkammer das Identifizieren des größten geschlossenen Blutmerkmals innerhalb einer vorbestimmten Region auf der Seite der linken Herzkammer, wo sich der Blutpool der rechten Herzkammer befinden soll, umfasst.

9. Verfahren nach einem der vorhergehenden Ansprüche, wobei der Schwellwert auf einem Mittel der Voxelwerte im Blutpool der linken Herzkammer basiert, wobei der Blutpool als die Region innerhalb der Kontur, die an die endokardiale Begrenzung angesetzt ist, identifiziert ist.

10. Datenanalyseeinheit (60) zum Detektieren eines Herzmuskeleisengehalts unter Verwendung von Schwarzblut-Magnetresonanzbildgebungsdaten, das Folgendes umfasst:

eine Eingabeeinheit (64), die dazu ausgelegt ist, die Bildgebungsdaten zu empfangen;
eine Datenverarbeitungseinheit (68), die dazu ausgelegt ist, die folgenden Schritte durchzuführen:

Ansetzen von Konturen (S2) an die epikardiale und die endokardiale Begrenzung der linken Herzkammer;
Erstellen einer binären Karte (S3) durch Segmentieren von Voxeln der Bildgebungsdaten davon abhängig, ob die Voxelwerte über oder unter einem Schwellwert liegen, wobei der Schwellwert ausgewählt wird, um zwischen Blut und Herzkammergewebe zu unterscheiden;
Verwenden der erstellten binären Karte, um den Abschnitt des Gewebes zwischen den angepassten Konturen, der dem Kammerseptum entspricht, zu identifizieren (S4); und
Detektieren eines Herzmuskeleisengehalts (S5) durch Durchführen von T2*-Messungen an einem Abschnitt der Bildgebungsdaten, der dem identifizierten Kammerseptum entspricht,

wobei der Schritt des Ansetzens von Konturen Folgendes umfasst:

einen Initialisierungsschritt (S21), bei dem zwei Kreise an die Bildgebungsdaten angesetzt werden, wobei ein erster der Kreise an die epikardiale Begrenzung der linken Herzkammer angesetzt wird und ein zweiter der Kreise an die endokardiale Begrenzung der linken Herzkammer angesetzt wird; und
einen Anpassungsschritt (S22), bei dem die angesetzten Kreise iterativ angepasst werden, um an den jeweiligen Begrenzungen der linken Herzkammer anzusitzen,

und wobei der Anpassungsschritt (S22) die folgenden Schritte umfasst:

einen ersten Anpassungsschritt, der das iterative Anpassen der angesetzten Kreise umfasst, um erste geschätzte Konturen (30) der linken Herzkammer zu erhalten;
einen zweiten Anpassungsschritt, der das Ansetzen einer Ellipse an jede der ersten geschätzten Konturen (30), die im ersten Anpassungsschritt erhalten wurden, um dadurch erste geschätzte Ellipsen (32) zu erhalten, umfasst; und
einen dritten Anpassungsschritt, der das Erhalten von zweiten geschätzten Konturen (34) als die Begrenzungen des Abschnitts des Herzmuskels, der sowohl von den ersten geschätzten Konturen (30) als auch den ersten geschätzten Ellipsen (32) umschlossen ist, umfasst.

11. Datenanalyseeinheit (60) nach Anspruch 10, die ferner Folgendes umfasst:
eine Benutzerschnittstelle (70), die es einem Benutzer erlaubt, manuell mit der Datenanalyseeinheit (60) zu interagieren, wobei die Datenanalyseeinheit (60) dazu ausgelegt ist, es einem Benutzer zu erlauben, die Ergebnisse von einem oder mehreren der Schritte des Ansetzens von Konturen an die epikardiale und die endokardiale Begrenzung der linken Herzkammer, des Erstellens der binären Karte und des Verwendens der erstellten binären Karte, um das Kammerseptum zu identifizieren, unter Verwendung der Benutzerschnittstelle (70) anzupassen.

**12.** MRI-Maschine (62), die Folgendes umfasst:

ein Datenerfassungssystem (66) zum Erfassen von Schwarzblut-Magnetresonanzbildgebungsdaten und die Datenanalyseeinheit (60) nach Anspruch 10.

**13.** Computerprogramm zum Detektieren eines Herzmuskeleisengehalts unter Verwendung von Schwarzblut-Magnetresonanzbildgebungsdaten, wobei das Computerprogramm derart ist, dass, wenn es auf einem Computer ausgeführt wird, den Computer veranlasst, das Verfahren nach Anspruch 1 durchzuführen.

**Revendications**

1. Procédé informatisé de détection de teneur en fer myocardique à l'aide de données d'imagerie à résonance magnétique en sang noir, comprenant :

l'ajustement de contours (S2) aux bords épicardique et endocardique du ventricule gauche dans les données d'imagerie ;
la création d'une carte binaire (S3) par segmentation de voxels des données d'imagerie selon si les valeurs de voxel sont supérieures ou inférieures à un seuil, le seuil étant choisi de manière à distinguer entre sang et tissu ventriculaire ;
l'utilisation de la carte binaire créée et des contours ajustés pour identifier (S4) la partie du tissu entre les contours ajustés correspondant au septum interventriculaire ; et
la détection de teneur en fer myocardique (S5) par la réalisation de mesures T2* sur une partie des données d'imagerie correspondant au septum interventriculaire identifié,
l'étape d'ajustement de contours comprenant :

une étape d'initialisation (S21) dans laquelle deux cercles sont ajustés aux données d'imagerie, un premier des cercles étant ajusté au bord épicardique du ventricule gauche et un deuxième des cercles étant ajusté au bord endocardique du ventricule gauche ; et
une étape de réglage (S22) dans laquelle les cercles ajustés sont réglés de manière itérative pour correspondre aux bords respectifs du ventricule gauche,

et l'étape de réglage (S22) comprenant les étapes suivantes :

une première étape de réglage comprenant le réglage itératif des cercles ajustés pour obtenir de premiers contours estimés (30) du ventricule gauche ;
une deuxième étape de réglage comprenant l'ajustement d'une ellipse à chacun des premiers contours estimés (30) obtenus dans la première étape de réglage, permettant ainsi d'obtenir de premières ellipses estimées (32) ; et
une troisième étape de réglage comprenant l'obtention de deuxièmes contours estimés (34) tandis que les marges de la partie du myocarde qui est entourée par à la fois lesdits premiers contours estimés (30) et lesdites premières ellipses estimées (32).

2. Procédé selon la revendication 1, dans lequel les données d'imagerie sont traitées pour obtenir une carte de bords et l'ajustement des deux cercles aux données d'imagerie comprend l'ajustement des deux cercles à la carte de bords.

3. Procédé selon la revendication 1 ou 2, dans lequel l'ajustement des deux cercles utilise la transformée de Hough circulaire.

4. Procédé selon l'une quelconque des revendications 1 à 3, dans lequel l'étape de réglage (S22) utilise le modèle de Chan-Vese.

5. Procédé selon l'une quelconque des revendications, comprenant en outre :
une quatrième étape de réglage comprenant le déplacement d'un deuxième contour estimé le plus externe (34) vers l'intérieur et un deuxième contour estimé le plus interne (34) vers l'extérieur d'une distance prédéterminée ou d'un nombre de pixels prédéterminé.

**6.** Procédé selon la revendication 5, dans lequel le nombre prédéterminé de pixels est un pixel.

**7.** Procédé selon l'une quelconque des revendications précédentes, dans lequel l'étape d'utilisation de la carte binaire créée (S4) pour identifier le septum interventriculaire comprend :

l'identification du pool sanguin du ventricule droit depuis la carte binaire ;
la détermination des deux angles différents auxquels une simple intersection se produit entre une ligne radiale s'étendant depuis le centre du ventricule gauche (22) et le pool sanguin identifié (17) du ventricule droit ; et
l'identification du septum interventriculaire tandis que la région à l'intérieur des contours ajustés identifiant les bords épicardique et endocardique et les deux lignes radiales s'étendant au niveau des deux angles déterminés.

**8.** Procédé selon la revendication 7, dans lequel l'identification du pool sanguin du ventricule droit comprend l'identification de la caractéristique sanguine fermée à l'intérieur d'une région prédéterminée sur le côté du ventricule gauche où on s'attend à trouver le pool sanguin du ventricule droit.

**9.** Procédé selon l'une quelconque des revendications précédentes, dans lequel le seuil est fonction d'une moyenne de valeurs de voxel dans le pool sanguin dans le ventricule gauche, le pool sanguin étant identifié comme région à l'intérieur du contour ajusté au bord endocardique.

**10.** Unité d'analyse de données (60) destinée à détecter une teneur en fer myocardique à l'aide de données d'imagerie à résonance magnétique en sang noir, comprenant :

une unité d'entrée (64) conçue pour recevoir les données d'imagerie ;
une unité de traitement de données (68) conçue pour effectuer les étapes suivantes :

l'ajustement de contours (S2) aux bords épicardique et endocardique du ventricule gauche ;
la création d'une carte binaire (S3) par segmentation de voxels dans les données d'imagerie selon si les valeurs de voxel sont supérieures ou inférieures à un seuil, le seuil étant choisi de manière à distinguer entre sang et tissu ventriculaire ;
l'utilisation de la carte binaire créée pour identifier (S4) la partie du tissu entre les contours ajustés correspondant au septum interventriculaire ; et
la détection d'une teneur en fer myocardique (S5) par la réalisation de mesures T2* sur une partie des données d'imagerie correspondant au septum interventriculaire identifié,

l'étape d'ajustement de contours comprenant :

une étape d'initialisation (S21) dans laquelle deux cercles sont ajustés aux données d'imagerie, un premier des cercles étant ajusté au bord épicardique du ventricule gauche et un deuxième des cercles étant ajusté au bord endocardique du ventricule gauche ; et
une étape de réglage (S22) dans laquelle les cercles ajustés sont réglés de manière itérative pour correspondre aux bords respectifs du ventricule gauche,

et l'étape de réglage (S22) comprenant les étapes suivantes :

une première étape de réglage comprenant le réglage itératif des cercles ajustés pour obtenir de premiers contours estimés (30) du ventricule gauche ;
une deuxième étape de réglage comprenant l'ajustement d'une ellipse à chacun des premiers contours estimés (30) obtenus dans la première étape de réglage, permettant ainsi d'obtenir de premières ellipses estimées (32) ; et
une troisième étape de réglage comprenant l'obtention de deuxièmes contours estimés (34) tandis que les bords de la partie du myocarde qui est entourée par à la fois lesdits premiers contours estimés (30) et lesdites premières ellipses estimées (32).

**11.** Unité d'analyse de données (60) selon la revendication 10, comprenant en outre :
une interface utilisateur (70) permettant à un utilisateur d'interagir manuellement avec l'unité d'analyse de données (60), l'unité d'analyse de données (60) étant conçue pour permettre à un utilisateur de régler les résultats d'au moins une des étapes d'ajustement de contours aux bords épicardique et endocardique du ventricule gauche, créant la carte binaire et utilisant la carte binaire créée pour identifier le septum interventriculaire, à l'aide de l'interface utilisateur (70).

12. Machine d'IRM (62), comprenant :

un système d'acquisition de données (66) destiné à acquérir des données d'imagerie à résonance magnétique en sang noir ; et
l'unité d'analyse de données (60) selon la revendication 10.

13. Programme informatique destiné à détecter une teneur en fer myocardique à l'aide de données d'imagerie à résonance magnétique en sang noir, le programme informatique étant tel que lorsqu'il est exécuté sur un ordinateur il amène l'ordinateur à effectuer le procédé selon la revendication 1.

Fig.1

S1 — Obtain myocardial black-blood MRI data

S2

S21 — Fit circles to epicardium and endocardium

S22 — Adapt circles towards local contour geometry

S23 — Exclude papillary muscles and/or burrs

S3 — Create binary map

S4 — Identify IS

S5 — Obtain MIC

Fig.2

Fig.3

Fig.4

Fig.5

Fig.6

Fig.7

Fig.8

Fig.9

Fig.10

Fig.11

Fig.12

Fig.13

Fig.14

Fig.15

Fig.16

Fig.17

Fig.18

Fig.19

Fig.20

Fig.21

## Fig.22

**Intra-observer (Manual)**

## Fig.23

**Inter-observer (Manual)**

## Fig.24

Intra-observer (sAISS)

## Fig.25

Inter-observer (sAISS)

Fig.26

sAISS vs. Manual

Fig.27

sAISS vs. AISS

Fig 28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006134430 A2
- US 2004153128 A1

**Non-patent literature cited in the description**

- **YANQIU FENG et al.** In vivo comparison of myocardial T1 with T2 and T2* in thalassaemia major. *Journal of Magnetic Resonance Imaging,* 31 January 2013, vol. 38 (3), 588-593
- *Thalassaemiatools - T2* assessment for the heart and liver,* 23 July 2010, http://web.archive.org/web/20100723144115/http://www.cmrtools.com/cmrTools/public /thalassaemiatools.pdf
- Cardiac T2* measurements in patients with iron overload: a comparison of imaging parameters and analysis techniques. **PHALLA OU et al.** MAGNETIC RESONANCE IMAGING. ELSEVIER SCIENCE, 03 December 2011, vol. 30, 641-648
- The shape analysis of ventricular septum based on medical images. **ZHANG YAONAN et al.** 2013 IEEE INTERNATIONAL CONFERENCE ON MEDICAL IMAGING PHYSICS AND ENGINEERING. IEEE, 19 October 2013, 201-206
- Gaussian Regularizing CV Model Using Entropy and Neighborhood Information. **ZHENG Q ; LU Z ; ZHANG M ; FENG Q ; CHEN W.** In: Proceedings of IFMBE, 2012. Springer, 2013, 1832-1835
- **PENNELL DJ.** T2* magnetic resonance: iron and gold. *JACC Cardiovasc Imaging,* 2008, vol. 1 (5), 579-581
- **TANNER MA ; HE T ; WESTWOOD MA ; FIRMIN DN ; PENNELL DJ.** Multi-center validation of the transferability of the magnetic resonance T2* technique for the quantification of tissue iron. *Haematologica,* 2006, vol. 91 (10), 1388-1391
- **DAAR S ; PATHARE AV ; JAIN R ; ZADJALI SA ; PENNELL DJ.** T2* cardiovascular magnetic resonance in the management of thalassemia patients in Oman. *Haematologica,* 2009, vol. 94 (1), 140-141
- **SMITH GC ; CARPENTER JP ; HE T ; ALAM MH ; FIRMIN DN ; PENNELL DJ.** Value of black blood T2* cardiovascular magnetic resonance. *J Cardiovasc Magn Reson,* 2011, vol. 13, 21
- **HE T ; GATEHOUSE PD ; KIRK P et al.** Black-blood T2* technique for myocardial iron measurement in thalassemia. *J Magn Reson Imaging,* 2007, vol. 25 (6), 1205-1209
- **YAMAMURA J ; GROSSE R ; GRAESSNER J ; JANKA GE ; ADAM G ; FISCHER R.** Distribution of cardiac iron measured by magnetic resonance imaging (MRI)-R*2. *J Magn Reson Imaging,* 2010, vol. 32 (5), 1104-1109
- **SAIVIROONPOM P ; VIPRAKASIT V ; BOONYASIRINANT T ; KHUHAPINANT A ; WOOD JC ; KRITTAYAPHONG R.** Comparison of the region-based and pixel-wise methods for cardiac T2* analysis in 50 transfusion-dependent Thai thalassemia patients. *J Comput Assist Tomo,* 2011, vol. 35 (3), 375-381
- **ILLINGWORTH J ; KITTLER J.** The adaptive hough transform. *IEEE Trans Pattern Anal Mach Intell,* 1987, vol. 9 (5), 690-698
- **CHUANG KS ; TZENG HL ; CHEN S ; WU J ; CHEN T.** Fuzzy c-means clustering with spatial information for image segmentation. *Comput Med Imag Grap,* 2006, vol. 30 (1), 9-15
- **CHAN TF ; VESE LA.** Active contours without edges. *IEEE Trans Image Process,* 2001, vol. 10 (2), 266-277
- **JONASSON L ; BRESSON X ; THIRAN JP ; WEDEEN VJ ; HAGMANN P.** Representing diffusion MRI in 5-D simplifies regularization and segmentation of white matter tracts. *IEEE Trans Med Imaging,* 2007, vol. 26 (11), 1547-1554
- **MASKA M ; DANEK O ; GARASA S ; ROUZAUT A ; MUNOZ-BARRUTIA A ; ORTIZ-DE-SOLORZANO C.** Segmentation and shape tracking of whole fluorescent cells based on the Chan-Vese model. *IEEE Trans Med Imaging,* 2013, vol. 32 (6), 995-1006
- **ABDOLI M ; DIERCKX RA ; ZAIDI H.** Contourlet-based active contour model for PET image segmentation. *Med Phys,* 2013, vol. 40 (8), 082507
- **LEE SH ; SEO JK.** Level set-based bimodal segmentation with stationary global minimum. *IEEE Trans Image Process,* 2006, vol. 15 (9), 2843-2852

- **OSHER S ; SETHIAN JA.** Fronts propagating with curvature-dependent speed: algorithms based on Hamilton-Jacobi formulations. *J Comput Phys,* 1988, vol. 79 (1), 12-49
- **AUBERT G ; KORNPROBST P.** Mathematical problems in image processing: partial differential equations and the calculus of variations. Springer, 2006
- **HE T ; ZHANG J ; CARPENTER JP et al.** Automated truncation method for myocardial T2* measurement in thalassemia. *J Magn Reson Imaging,* 2013, vol. 37 (2), 479-483
- **MARQUARDT DW.** An algorithm for least-squares estimation of nonlinear parameters. *J Soc Ind Appl Math,* 1963, vol. 11 (2), 431-441
- **MARTIN BLAND J ; ALTMAN D.** Statistical methods for assessing agreement between two methods of clinical measurement. *Lancet,* 1986, vol. 327 (8476), 307-310